(19) Europäisches Patentamt
European Patent Office
Office européen des brevets

(11) EP 0 498 729 B1

(12) # FASCICULE DE BREVET EUROPEEN

(45) Date de publication et mention de la délivrance du brevet:
**31.01.1996 Bulletin 1996/05**

(51) Int Cl.$^6$: **C07K 5/062**, A61K 7/48

(21) Numéro de dépôt: **92400306.4**

(22) Date de dépôt: **06.02.1992**

(54) **Dérivés de dipeptides à groupement uréthanne, leur préparation et leur application, notamment comme agents hydratants et comme agents tensioactifs dans des compositions cosmétiques, pharmaceutiques ou alimentaires**

Dipeptidderivate mit Urethan-Gruppierung, deren Herstellung und Anwendung, insbesondere als hydrierende und tensioaktive Wirkstoffe bei kosmetischen, pharmazeutischen und Nahrungszubereitungen

Dipeptide derivatives with urethan groupings, their preparation and their application, especially as hydratating agents and tensioactive agents in cosmetic, pharmaceutical and alimentary preparations

(84) Etats contractants désignés:
**AT BE CH DE DK ES FR GB GR IT LI NL PT SE**

(30) Priorité: **06.02.1991 FR 9101309**

(43) Date de publication de la demande:
**12.08.1992 Bulletin 1992/33**

(73) Titulaire: **L'OREAL**
**F-75008 Paris (FR)**

(72) Inventeur: **Philippe, Michel**
**F-92160 Antony (FR)**

(74) Mandataire: **Tonnellier, Jean-Claude et al**
**F-75008 Paris (FR)**

(56) Documents cités:
**EP-A- 0 203 418** **EP-A- 0 408 448**

- **patent abstracts of japan, vol.12, no 349 (C-529)[3196], 20 septembre 1988;**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).



Printed by Jouve, 75001 PARIS (FR)

## Description

Dérivés de dipeptides à groupement uréthanne, leur préparation et leur application, notamment comme agents hydratants et comme agents tensioactifs dans des compositions cosmétiques, pharmaceutiques ou alimentaires.

La présente invention a pour objet de nouveaux dérivés de dipeptides à groupement uréthanne, leur préparation et leur application, notamment comme agents hydratants ou comme agents tensioactifs doux dans des compositions cosmétiques, hygiéniques, pharmaceutiques ou alimentaires.

Des dérivés de dipeptides à groupement amide, et leur utilisation comme inhibiteurs de la prolifération cellulaire ou comme détergents, sont décrits dans les demandes de brevet JP-84994/1984 et JP-146851/1988.

On a maintenant découvert que des dérivés N-alkoxycarbonylés à longue chaîne de certains dipeptides ont des propriétés tensioactives supérieures à celles des lipoglycines à fonction amide correspondantes, et sont utilisables en outre comme agents hydratants pour la peau.

L'invention a plus précisément pour objet les dérivés d'uréthanne de formule générale I :

$$R'O\text{-}CO\text{-}NH\text{-}CH_2\text{-}CO\text{-}NH\text{-}CH(R)\text{-}COOH \qquad (I)$$

dans laquelle R représente un groupement -H ou un groupement $-CH_2OH$,

et R' représente un groupement alkyle linéaire ou ramifié, éventuellement insaturé, ayant de 8 à 16 atomes de carbone,

ainsi que les sels des dérivés de formule I, et les mélanges des dérivés de formule I et/ou de leurs sels.

Les composés de formule I pour lesquels R représente $-CH_2OH$ peuvent dériver de la L-, D- ou D,L-sérine.

Dans les composés de formule I, R' représente notamment un groupement alkyle ayant 8 à 16 atomes de carbone, éventuellement mono- ou poly-insaturé, linéaire ou ramifié.

Parmi les sels des composés de formule I (carboxylates), on citera les sels compatibles avec l'application sur la peau, et notamment les sels métalliques tels que les sels de sodium, de zinc, de magnésium, les sels d'aluminium et les sels cuivriques ou les sels de cations organiques tels que les sels d'un ammonium quaternaire de formule Ia :

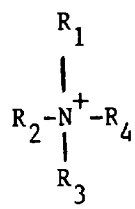

(Ia)

dans laquelle $R_1$, $R_2$, $R_3$ et $R_4$ représentent indépendamment un groupement $-CH_3$, $-CH_2\text{-}C_6H_5$ ou $-CH_2\text{-}CH_2OH$.

Par convention, l'expression "composés" ou "dérivés" de formule I désignera ci-après les composés de formule I et/ou leur sels.

L'invention a également pour objet un procédé de préparation des composés de formule I.

Ce procédé est caractérisé par le fait que :

a) soit on fait réagir un composé de formule III :

$$R'O\text{-}CO\text{-}NH\text{-}CH_2\text{-}CO\text{-}O\text{-}CO\text{-}OR'' \qquad (III)$$

dans laquelle R' est défini comme précédemment et R" représente un groupement éthyle ou isopropyle, avec un sel de glycine ou de sérine pour former un dérivé de formule I correspondant ;

b) soit on fait réagir un sel de diglycine avec un chloroformiate de formule IV :

$$R'\text{-}O\text{-}CO\text{-}Cl \qquad (IV)$$

pour former un dérivé de formule I correspondant (avec R=H) ;

c) et que, si désiré, on transforme selon les méthodes connues ledit dérivé de formule I obtenu en sel correspondant.

Dans le cas où l'on fait réagir le composé de formule III avec un sel de glycine, on obtient un composé de formule I correspondant, avec R=H. Dans le cas où l'on fait réagir le composé de formule III avec un sel de D-,L- ou (D,L)-sérine, on obtient un composé de formule I correspondant, avec R = $CH_2OH$.

Les composés de formule III peuvent eux-mêmes être obtenus par une réaction d'un composé de formule R"O-CO-Cl avec un sel d'un composé de formule (V) :

$$R'O\text{-}CO\text{-}NH\text{-}CH_2\text{-}COOH \qquad (V).$$

Le sel de départ de glycine, de diglycine ou de sérine, ou le sel du composé de formule (V), est par exemple un sel de métal alcalin ou un sel d'amine telle que la triéthylamine.

La réaction entre le sel d'acide aminé et le dérivé de formule III peut être effectuée par exemple dans un solvant classique tel qu'un mélange eau/N,N-diméthylformamide ou eau/tétrahydrofuranne, généralement à la température ambiante.

La réaction entre le sel de diglycine et le chloroformiate de formule IV peut être effectuée à la température ambiante dans les mêmes solvants.

Les dérivés d'uréthanne de l'invention ont des propriétés tensio-actives.

L'invention a donc notamment pour objet l'utilisation des composés de formule I, et de leurs sels, comme agents tensioactifs, en particulier dans des compositions cosmétiques, hygiéniques ou dermopharmaceutiques, ou encore dans des compositions détergentes en général, par exemple des détergents ménagers. Les composés de formule I et leurs sels possèdent notamment un pouvoir moussant très important, et un pouvoir détergent élevé, supérieur à celui de la N-dodécanoyl-glycyl-glycine décrite dans le brevet JP-84994/1984, comme cela est montré dans la partie expérimentale ci-après.

Les composés de formule I sont utilisables comme détergents dans des milieux ayant un pH de 7 à 13 et en particulier de 7 à 9.

Ils peuvent être utilisés notamment comme détergents doux dans des compositions présentant un pouvoir moussant, (compositions cosmétiques, hygiéniques ou pharmaceutiques pour la peau ou les cheveux, ou pour l'hygiène bucco-dentaire ou encore comme émulsifiants dans des préparations alimentaires.

Certains composés de formule I et leurs sels peuvent aussi être utilisés chez l'homme comme agents d'hydratation de la peau, capables notamment de diminuer la perte en eau de la peau. Ces composés, par exemple la N-dodécyloxycarbonyl glycyl-(D,L) sérine, permettent donc notamment de conserver ou de restaurer la souplesse de la peau, son élasticité et sa fonction de barrière à l'entrée des substances toxiques. On sait que les compositions cosmétiques ou dermopharmaceutiques destinées à hydrater la peau (préparations hydratantes) sont utilisées chez les personnes ayant une peau dite sèche. Ce phénomène est caractérisé généralement par une peau ayant un taux d'évaporation nettement plus élevé que celui d'une peau saine, par une perte de l'élasticité cutanée et par la formation de rides. Il peut être provoqué notamment par des troubles pathologiques de la kératinisation, par le vieillissement ou par l'exposition excessive au soleil ou à divers agents extérieurs (détergents usuels, savons, solvants, atmosphère sèche, etc...). Ce phénomène peut affecter toutes les parties du corps, et particulièrement le visage, le cou et les mains.

En outre, certains dérivés de formule (I) peuvent former dans l'eau ou dans les solvants aqueux des structures vésiculaires capables de piéger et de retenir des substances hydrophobes ou hydrophiles, et peuvent être utilisés sous cette forme comme véhicules d'ingrédients actifs lipophiles ou hydrophiles, notamment dans des compositions cosmétiques, hygiéniques ou pharmaceutiques.

La présente invention a donc également pour objet une composition cosmétique, hygiénique ou pharmaceutique caractérisée par le fait qu'elle comprend comme ingrédient actif au moins un dérivé de formule I, tel que défini précédemment, ou un sel correspondant, dans un véhicule compatible avec l'application chez l'homme sur la peau et/ou sur les cheveux et/ou avec l'application dans les soins d'hygiène bucco-dentaire.

Dans les compositions de l'invention, les dérivés de formule I, sont présents à une concentration pouvant aller de 0,05 à 20 %, et de préférence de 0,5 à 10% en poids, par rapport au poids total de la composition.

Les compositions de l'invention sont notamment des solutions du type lotions, moussantes ou non ; des émulsions de consistance liquide ou semi-liquide du type laits, obtenues par dispersion d'une phase grasse dans une phase aqueuse ou inversement ; des suspensions ou émulsions de consistance molle du type crème ou pommade ; des gels ; ou encore des préparations solides telles que des sticks, des pains de nettoyage ou des tampons imprégnés.

Les véhicules présents dans les compositions de l'invention sont les véhicules classiques utilisés dans ce genre de composition. Il s'agit par exemple de l'eau et des solvants organiques compatibles avec l'application cutanée tels que par exemple l'acétone, l'alcool isopropylique, l'alcool éthylique, les triglycérides d'acides gras en $C_6$-$C_{24}$, les éthers de glycols tels que les éthers d'alkyle inférieur de mono-ou di-alkylène glycols, l'alkylène ayant par exemple 2 à 4 atomes de carbone. On peut également utiliser comme solvants les esters de polyalkylèneglycol et d'acides à chaîne courte en $C_1$-$C_4$, ou encore des silicones volatiles.

Les compositions peuvent aussi contenir, le cas échéant, des corps gras, notamment des huiles naturelles ou synthétiques.

Les compositions de l'invention peuvent également renfermer des agents épaississants ou gélifiants tels que la cellulose ou des dérivés de cellulose, par exemple à raison de 0,5 à 20% en poids par rapport au poids total de la composition. Les agents épaississants peuvent encore être constitués par des polymères acryliques, des alginates, des gommes, telles que la gomme de xanthane, de guar, de caroube, la gomme arabique ou bien des polyéthylèneglycols, des bentonites et des montmorillonites.

Les compositions de l'invention peuvent en outre contenir d'autres agents hydratants ou humectants connus, tels que la glycérine, la triacétine, ou plus généralement d'autres ingrédients actifs tels que des agents actifs contre le

vieillissement de la peau.

Les compositions de l'invention peuvent également contenir des adjuvants usuels tels que des agents anti-oxydants, des agents conservateurs, des parfums, des colorants, etc...

Parmi les anti-oxydants, on citera les tert-butylhydroxyquinone, le butylhydroxyanisole, le butylhydroxytoluène et l'alpha-tocophérol et ses dérivés.

Les compositions pour la peau se présentent notamment sous la forme de crèmes, de laits, de gels, de lotions éventuellement épaissies, de solutions moussantes pour la douche ou le bain, de tampons imprégnés, de pommades, de sticks ou sous forme de pains ou de masques hydratants.

Les compositions pour cheveux sont notamment des shampooings dans lesquels le dérivé de formule I ou le sel correspondant, peut être associé à d'autres agents tensio-actifs tels que les tensio-actifs anioniques, cationiques, non ioniques, amphotères ou leurs mélanges.

Les compositions de l'invention peuvent également se présenter sous la forme de solutions ou de dispersions contenant des dérivés de formule I tels que définis ci-dessus, sous la forme vésiculaire, les vésicules pouvant alors servir d'agents d'encapsulation pour des ingrédients actifs lipophiles ou hydrophiles tels que l'acide rétinoïque, des agents filtrant les rayons ultraviolets, ou encore d'autres agents hydratants.

Toutes ces compositions sont préparées selon les méthodes usuelles.

Les compositions de l'invention peuvent être également des compositions pour les soins d'hygiène bucco-dentaire, dans lesquelles les composés de formule I et/ou leurs sels jouent notamment le rôle d'agents nettoyants et moussants. L'un des intérêts de l'utilisation de ces composés dans les compositions de ce type est leur absence de nocivité. Ces compositions sont par exemple des bains de bouche ou des dentifrices. Les dentifrices peuvent se présenter sous la forme de pâtes ou sous la forme de gels transparents. Ils contiennent, outre au moins un agent tensioactif selon l'invention, au moins un matériau minéral pulvérulent jouant le rôle d'agent de polissage, par exemple une poudre d'alumine ou de silice. L'agent de polissage représente par exemple de 10 à 80 % en poids par rapport au poids total de la composition. Les compositions dentifrices peuvent contenir en outre des agents de cohésion comme des gommes naturelles ou des épaississants synthétiques (notamment des dérivés de cellulose tels que la méthylcellulose, les hydroxyalkylcelluloses ou le sel de sodium de la carboxyméthylcellulose). Ces agents de cohésion peuvent être incorporés par exemple dans une proportion pondérale pouvant aller jusqu'à 10 %.

Les compositions dentifrices peuvent êgalement contenir des agents de texture et de consistance, dans une proportion pondérale pouvant aller par exemple jusqu'à 60 %. On utilise par exemple comme agent de texture le sorbitol, qui a par ailleurs des propriétés édulcorantes et anti-bactériennes.

Les compositions sous forme de bains de bouche sont des compositions liquides qui consistent essentiellement en une solution aqueuse d'un agent tensioactif nettoyant et moussant. Outre l'agent tensioactif selon l'invention, ces compositions peuvent encore contenir un ou plusieurs ingrédients usuels tels que des agents épaississants.

Les compositions d'hygiène bucco-dentaire, quelle que soit leur présentation, peuvent contenir en outre, en proportion efficace, au moins un ingrédient usuel choisi parmi les édulcorants, les agents aromatisants, les agents antibactériens, les sources d'ions fluorure, les agents conservateurs, etc...

La préparation des compositions dentifrices ou des bains de bouche, ainsi que les ingrédients utilisables, sont bien connus et décrits par exemple dans les ouvrages suivants : Handbook of Cosmetic Science, H.W. Hibbott Ed. Pergamon Press (Oxford, Londres, Nex-York, Paris), et Harry's Cosmeticology, Leonard Hill Books (Londres).

L'invention a également pour objet l'utilisation d'un dérivé de formule (I) tel que défini précédemment, comme agent tensioactif et / ou comme agent hydratant dans la préparation d'une composition cosmétique, hygiénique ou pharmaceutique destinée au traitement ou au soin des peaux sèches.

L'invention a en outre pour objet un procédé de traitement cosmétique destiné notamment à améliorer l'aspect et l'élasticité de la peau des personnes à peau sèche, ou destiné à prévenir l'apparition des troubles esthétiques provoqués par ce phénomène de peau sèche, caractérisé par le fait que l'on applique sur la peau des parties du corps concernées, y compris éventuellement le cuir chevelu, une composition cosmétique telle que définie précédemment.

L'application des compositions de l'invention est effectuée selon les méthodes usuelles.

Le procédé de traitement cosmétique de l'invention est applicable en complément au traitement de dermatoses sèches, d'ichtiosis, de xéroses, etc....

Les exemples suivants illustrent l'invention sans toutefois la limiter :

EXEMPLES DE PREPARATION

Tous les lipodipeptides à fonction uréthanne, de formule I, décrits ci-après, sont synthétisés selon le mode opératoire suivant :

A. Préparation de l'alkoxycarbonyl-glycine

Le sel de sodium de la glycine est préparé par addition d'un équivalent d'hydroxyde de sodium (solution aqueuse à 10 %) à un équivalent de glycine. Le mélange est dilué ensuite par le tétrahydrofuranne jusqu'à une concentration en sel de sodium de 20 %. A ce mélange, on ajoute 1 équivalent du chloroformiate choisi, dilué à 25 % dans du tétrahydrofuranne, tout en prenant soin de maintenir le pH du milieu réactionnel supérieur à 9 par addition d'une solution d'hydroxyde de sodium à 10 %. Le mélange est ensuite laissé sous agitation pendant 3 heures, puis on verse de l'acide chlorhydrique concentré jusqu'à obtention d'un pH égal à 2. Le milieu ainsi acidifié est extrait par un mélange eau/acétate d'éthyle (1 : 1). Les phases organiques réunies sont séchées et concentrées sous pression réduite, puis le résidu est recristallisé dans un solvant choisi parmi l'heptane, l'éther iso-propylique, le tert-butylméthyl éther, l'acétate d'éthyle ou leurs mélanges.

B. Préparation du lipodipeptide de formule (I)

1er Procédé :

A 1 équivalent d'alkoxycarbonyl-glycine mis en solution à 30 % dans le tétrahydrofuranne, on ajoute 1 équivalent de triéthylamine. Le milieu résultant est agité 1 heure à température ambiante puis additionné à une solution de chloroformiate d'éthyle ou d'isopropyle (1 éq.) à 10 % dans le tétrahydrofuranne, en maintenant la température à - 10°C. Le mélange est ensuite agité pendant 3 heures à température ambiante, puis filtré et le filtrat est versé dans une solution de sel de sodium de glycine (ou sérine) tout en maintenant le pH supérieur à 9.

Le milieu réactionnel est alors laissé à température ambiante sous agitation pendant 3 heures, puis acidifié par de l'acide chlorhydrique concentré jusqu'à l'obtention d'un pH égal à 2, et enfin extrait à l'aide d'un mélange eau/acétate d'éthyle (1 : 1). Les phases organiques réunies sont séchées et concentrées sous pression réduite et le résidu est recristallisé dans un solvant choisi parmi l'eau, l'heptane, l'éther isopropylique, le tert-butylméthyléther, l'acétate d'éthyle ou leurs mélanges.

2ème procédé :

Le sel de sodium de la glycylglycine est préparé par addition d'un équivalent d'hydroxyde de sodium (solution aqueuse à 10 %) à 1 équivalent de glycylglycine. On dilue ensuite au tétrahydrofuranne pour que le sel de sodium ait finalement une concentration de 10 % dans le mélange.

A ce mélange est ajouté 1 équivalent de chloroformiate d'alkyle en solution, à une concentration de 25 %, dans le tétrahydrofuranne.

Pendant l'addition le pH du milieu réactionnel est conservé supérieur à 9 par addition d'une solution d'hydroxyde de sodium à 10 %.

Après addition, le milieu réactionnel est laissé à température ambiante sous agitation pendant 3 heures tout en surveillant le pH.

On acidifie alors le milieu réactionnel par l'acide chlorhydrique concentré jusqu'à obtention d'un pH voisin de 2. On obtient un précipité.

Ce précipité est essoré, lavé à l'eau et séché.

C. Préparation des sels de lipodipeptide (par exemple sel de sodium ou de triéthanolamine).

Ces sels sont obtenus par une méthode usuelle consistant à additionner 1 équivalent d'hydroxyde de sodium (solution aqueuse à 10 %), ou 1 équivalent de triéthanolamine, à une solution du lipodipeptide à 5 % dans un mélange isopropanol/eau (2 : 1) à une température pouvant varier par exemple entre 20 et 65°C. La solution est ensuite concentrée à sec pour obtenir le sel attendu.

EXEMPLE 1 : Préparation de la N-dodécyloxycarbonyl-glycyl-(D,L) sérine

Ce produit est obtenu selon le procédé décrit ci-dessus. Il est recristallisé dans un mélange acétate d'éthyle/heptane.

F = 104°C

Analyse élémentaire $C_{18}H_{34}N_2O_6$ ; M = 374,5

| | C | H | N |
|---|---|---|---|
| Calc.% | 57,73 | 9,15 | 7,48 |
| Tr.% | 58,00 | 9,11 | 7,44 |

Le spectre R.M.N du $^{13}C$ est conforme à la structure indiquée.

EXEMPLE 2 : Préparation de la N-dodécyloxycarbonyl-glycyl-glycine

Ce produit est obtenu selon le procédé décrit ci-dessus. Il est recristallisé dans un mélange acétate d'éthyle/heptane.
F : 145°C
Analyse élémentaire : $C_{17}R_{32}N_2O_5$ ; M = 344,5

| | C | H | N |
|---|---|---|---|
| Calc.% | 59,28 | 9,36 | 8,13 |
| Tr.% | 59,14 | 9,56 | 8,42 |

Le spectre de R.M.N. du $^{13}C$ est conforme à la structure indiquée.

EXEMPLE 3: Préparation du sel de triéthanolamine de N-dodécyloxycarbonyl-glycyl-(D,L) sérine

Ce produit est obtenu selon le procédé décrit ci-dessus sous forme d'une pâte jaune.
Analyse élémentaire $C_{24}H_{49}N_3O_9$, $1H_2O$ ; M = 541,7

| | C | H | N |
|---|---|---|---|
| Calc.% | 53,17 | 9,49 | 7,76 |
| Tr.% | 53,20 | 9,33 | 8,15 |

EXEMPLE 4: Préparation du sel de sodium de la N-dodécyloxycarbonyl glycyl-(D,L) sérine

Ce sel est obtenu selon le procédé décrit ci-dessus sous forme d'un solide blanc.
F = 96°C
Analyse élémentaire : $C_{18}H_{33}N_2NaO_6$ ; M = 396,5

| | C | H | N |
|---|---|---|---|
| Calc.% | 54,53 | 8,39 | 7,07 |
| Tr.% | 54,59 | 8,50 | 7,14 |

EXEMPLE 5: Préparation du sel de sodium du N-dodécyloxycarbonyl-glycyl-glycine

Ce sel est obtenu selon le procédé décrit ci-dessus sous forme d'une pâte blanche.

Analyse élémentaire : $C_{17}H_{31}N_2NaO_5$, 3,5 $H_2O$ ; M = 429,5

| | C | H | N |
|---|---|---|---|
| Calc.% | 47,55 | 8,92 | 5,36 |
| Tr.% | 47,68 | 8,53 | 5,50 |

EXEMPLE 6 : Préparation de la N-éthyl-2-hexyloxycarbonyl-glycyl-glycine

Ce produit est obtenu selon un procédé analogue à celui décrit ci-dessus. Il est recristallisé dans un mélange acétate d'éthyle/méthanol.

F = 112°C.

Analyse élémentaire : $C_{13}H_{24}N_2O_5$ ; M = 288,4

| | C | H | N |
|---|---|---|---|
| Calc.% | 54,15 | 8,39 | 9,72 |
| Tr.% | 54,14 | 8,46 | 9,62 |

Le spectre RMN du $^{13}C$ est comforme à la structure indiquée.

EXEMPLE 7 : Préparation de la N-tétradécyloxycarbonyl-glycyl-glycine

Ce produit est obtenu selon un procédé analogue à celui décrit ci-dessus sous forme d'une poudre blanche.

F = 134°C

Analyse élémentaire : $C_{19}H_{36}N_2O_5$, 0,5 $H_2O$, M = 381,5

| | C | H | N |
|---|---|---|---|
| Calc.% | 59,81 | 9,77 | 7,34 |
| Tr.% | 59,97 | 9,52 | 7,35 |

Le sepctre RMN du $^{13}C$ est conforme à la structure indiquée.

EXEMPLE 8 : Préparation de la N-hexadécyloxycarbonyl-glycyl-glycine

Ce produit est obtenu selon un procédé analogue à celui décrit ci-dessus sous forme d'une poudre blanche.
F = 145°C
Analyse élémentaire : $C_{21}H_{40}N_2O_5$ ; M = 400,5

| | C | H | N |
|---|---|---|---|
| Calc.% | 62,97 | 10,07 | 6,99 |
| Tr.% | 62,51 | 10,13 | 6,96 |

Le sepctre RMN du $^{13}C$ est conforme à la structure indiquée.

EXEMPLES DE COMPOSITIONS COSMETIQUES

Dans ces exemples, les produits désignés par des marques commerciales sont les suivants :

KLUCEL H : Hydroxypropylcellulose, vendue par la société HERCULES.
TWEEN 60 : Monostéarate de sorbitan polyoxyéthyléné à 20 moles d'oxyde d'éthylène, vendu par la société ICI AMERICAS.
CARBOPOL 940 : Polymère acrylique réticulé avec un agent polyfonctionnel, vendu par la société GOODRICH.

EXEMPLE A: Lotion

On a préparé, par mélange des ingrédients ci-après, une lotion à activité hydratante.

| | |
|---|---|
| N-dodécyloxycarbonyl-glycyl-(D,L) sérine | 3,00 g |
| Isopropanol | 40,00 g |
| Anti-oxydant | 0,05 g |
| Conservateur | 0,3 g |
| Eau, q.s.p. | 100,00 g |

On applique cette lotion après le bain, éventuellement sur le corps entier, pour hydrater la peau.

EXEMPLE B : Gel

On a préparé, par mélange des ingrédients ci-après, une composition sous forme de gel à activité hydratante

| | |
|---|---|
| N-dodécyloxycarbonyl-glycyl-(D,L) sérine | 3,00 g |
| Klucel H | 1,00 g |
| Isopropanol | 40,00 g |
| Conservateur | 0,3 g |
| Anti-oxydant | 0,05 g |
| Eau, q.s.p. | 100,00 g |

On applique ce gel sur le visage et le cou après le nettoyage quotidien.

EXEMPLE C : Crème de soins (émulsion H/E) pour la peau.

Cette crème à la composition suivante :

| | |
|---|---|
| Sel de sodium de N-dodécyloxycarbonyl-glycyl-(D,L) sérine | 3,00 g |
| Stéarate de glycérol | 2,00 g |
| TWEEN 60 | 1,00 g |
| Alcool cétylique | 0,50 g |
| Acide stéarique | 1,40 g |
| Triéthanolamine | 0,70 g |
| CARBOPOL 940 (neutralisé par la triéthanolamine) | 0,40 g |
| Fraction liquide de graisse de karité | 12,00 g |
| Perhydrosqualène de synthèse | 12,00 g |
| Anti-oxydant | 0,05 g |
| Parfum | 0,50 g |
| Conservateur | 0,30 g |
| Eau, q.s.p | 100,00 g |

Cette crème est préparée de la façon suivante :

On ajoute le CARBOPOL 940 neutralisé par la triéthanolamine à une partie de l'eau (85 à 90 %) et on chauffe à 75-80°C. On ajoute alors, en agitant, la phase grasse (stéarate de glycérol, TWEEN 60, acide stéarique, alcool cétylique, fraction liquide de graisse de karité, perhydrosqualène, anti-oxydant) portée à la même température, dans laquelle on a ajouté en dernier lieu la triéthanolamine. Après 10 min d'agitation, on ajoute le dérivé dipeptidique et le conservateur préalablement versés dans le restant de l'eau. Au bout de 10 min supplémentaires, on ajoute le parfum puis on arrête l'agitation et on refroidit jusqu'à la température ambiante.

EXEMPLE D : Shampoing

| | |
|---|---|
| Sel de sodium de la N-dodécyloxycarbonyl-glycyl-(D,L) sérine | 6,0 g |
| Klucel H | 1,0 g |
| Parfum | 0,5 g |
| Conservateur | 0,3 g |
| Eau q.s.p. | 100,0 g |

Le sel de sodium de la N-dodécyloxy-carbonyl-glycyl-(D,L) sérine est dissous à 60°C dans l'eau jusqu'à obtention

d'une solution limpide. On ajoute à ce milieu à 60°C une solution aqueuse épaissie par le KLUCEL H elle-même à 60°C. On ajoute le parfum et le conservateur, on ajuste le poids final et on laisse refroidir.

Ce shampoing présente un bon pouvoir moussant.

EXEMPLE E :

On prépare une pâte dentifrice de composition suivante (% en poids)

| Ingrédients | % |
|---|---|
| Alumine en poudre | 52 |
| Carboxyméthyl cellulose | 1 |
| Lauroyl glycyl sérine neutralisée par la soude jusqu'à pH = 7 | 1 |
| Sorbitol à 70 % de matière active | 35 |
| Dioxyde de titane | 0,5 |
| Parahydroxybenzoyate de méthyle | 0,2 |
| Fluorure de sodium | 0,22 |
| Agent de sapidité q.s | |
| colorant q.s | |
| Edulcorant q.s | |
| Eau qsp | 100 |

ETUDE COMPARATIVE :

Pouvoir détergent

On évalue ce pouvoir détergent de la façon suivante : le test consiste à déposer sur de la laine une tache de sebum artificiel simplifié coloré au Noir Soudan, puis à effectuer un lavage à l'aide d'une machine à shampooing avec la solution à tester. La tache résiduelle est évaluée par rapport à une gamme étalon obtenue à l'aide de solutions de laurylsulfate de sodium à diverses concentrations (solutions de référence). Les résultats sont exprimés en pourcentage de laurylsulfate de sodium (concentration de la solution de référence présentant une tache identique).

Les composés de formule I sont étudiés sous la forme de solutions à 5 % (poids/volume) dans l'eau distillée.

Les résultats sont résumés dans le tableau I.

TABLEAU I

| Composé de l'exemple | équivalent de laurylsulfate de sodium (%) |
|---|---|
| 3 | 1,7 |
| 4 | 2,3 |
| 5 | 1,8 |
| Produit de comparaison | 1,5 |

Le sebum artificiel simplifié coloré au Noir de Soudan à la composition suivante :

| | |
|---|---|
| squalène | 19 % |
| cholestérol | 5 % |
| trioléïne | 43 % |
| acide oléïque | 31 % |
| Noir de Soudan | 2 % |
| | 100 % |

On applique cette composition de sebum artificiel en solution à 9 % (poids/ml) dans le chloroforme pour déposer la tache.

Le Noir de Soudan ou Solvent Black 3, porte la référence C.I.26150.

## Revendications

### Revendications pour les Etats contractants suivants : AT, BE, CH, LI, DE, DK, FR, GB, GR, IT, NL, SE, PT

**1.** Dérivés de dipeptide à groupement uréthanne de formule générale I :

$$R'O\text{-}CO\text{-}NH\text{-}CH_2\text{-}CO\text{-}NH\text{-}CH(R)\text{-}COOH \qquad (I)$$

dans laquelle R représente un groupement -H ou un groupement $-CH_2OH$,

et R' représente un groupement alkyle linéaire ou ramifié, éventuellement insaturé, ayant de 8 à 16 atomes de carbone,

ainsi que les sels des dérivés de formule I, et les mélanges des dérivés de formule I et/ou de leurs sels.

**2.** Dérivés selon la revendication 1, caractérisés par le fait que lesdits sels sont des sels métalliques ou des sels de cations organiques compatibles avec une application cosmétique, pharmaceutique ou alimentaire.

**3.** Dérivés selon la revendication 2, caractérisés par le fait que lesdits sels sont choisis parmi les sels de sodium, de zinc, de magnésium et d'aluminium, et les sels cuivriques.

**4.** Dérivés selon la revendication 2, caractérisés par le fait que lesdits sels sont des sels d'ammonium quaternaires.

**5.** Dérivés selon la revendication 4, caractérisés par le fait que lesdits sels d'ammonium quaternaires sont des sels d'un cation de formule

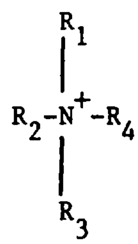

dans laquelle $R_1$, $R_2$, $R_3$ et $R_4$ représentent indépendamment un groupement $-CH_3$, $-CH_2-C_6H_5$ ou $-CH_2-CH_2OH$.

**6.** Dérivés selon l'une quelconque des revendications précédentes, 2caractérisés par le fait que R' est un groupement alkyle ayant 8 à 16 atomes de carbone.

**7.** Dérivés selon l'une quelconque des revendications précédentes, caractérisés par le fait qu'ils sont choisis parmi :

- la N-dodécyloxycarbonyl-glycyl-(D,L) sérine
- la N-dodécyloxycarbonyl-glycyl-glycine
- la N-éthyl-2-hexyloxycarbonyl-glycyl-glycine;
- la N-tétradécyloxycarbonyl-glycyl-glycine;
- la N-hexadécyloxycarbonyl-glycyl-glycine ;

ainsi que leurs sels.

**8.** Procédé de préparation d'un dérivé tel que défini dans l'une quelconque des revendications précédentes, caractérisé par le fait que :

a) soit on fait réagir un composé de formule III :

$$R'O\text{-}CO\text{-}NH\text{-}CH_2\text{-}CO\text{-}O\text{-}CO\text{-}OR'' \qquad (III)$$

dans laquelle R' est défini comme précédemment et R" représente un groupement éthyle ou isopropyle, avec un sel de glycine ou de sérine pour former un dérivé de formule I correspondant ;
b) soit on fait réagir un sel de diglycine avec un chloroformiate de formule IV :

$$R'\text{-}O\text{-}CO\text{-}Cl \qquad (IV)$$

pour former un dérivé de formule I correspondant (avec R=H) ;
c) et que, si désiré, on transforme selon les méthodes connues ledit dérivé de formule I obtenu en sel correspondant.

9. Utilisation d'au moins un dérivé de dipeptide, tel que défini dans l'une quelconque des revendications 1 à 7, comme agent tensioactif.

10. Composition cosmétique, hygiénique ou pharmaceutique, caractérisée par le fait qu'elle comprend comme ingrédient actif, notamment tensioactif et/ou hydratant, au moins un dérivé de dipeptide tel que défini dans l'une quelconque des revendications 1 à 7, dans un véhicule compatible avec l'administration sur la peau et/ou sur les cheveux, ou compatible avec l'application dans les soins d'hygiène bucco-dentaire.

11. Composition selon la revendication 10, caractérisée par le fait que la concentration dudit dérivé de dipeptide est dans la gamme de 0,05 à 20 % en poids, par rapport au poids total de la composition.

12. Composition selon la revendication 11, caractérisée par le fait que ladite concentration est dans la gamme 0,5 à 10 % en poids par rapport au poids total de la composition.

13. Utilisation d'un dérivé de dipeptide tel que défini dans l'une quelconque des revendications 1 à 7 comme ingrédient actif dans la préparation d'une composition cosmétique ou pharmaceutique destinée au traitement ou au soin des peaux sèches.

14. Procédé de traitement cosmétique destiné notamment à améliorer l'aspect et l'élasticité de la peau des personnes à peau sèche, ou destiné à prévenir l'apparition des troubles esthétiques provoqués par ce phénomène de peau sèche, caractérisé par le fait que l'on applique sur la peau des parties du corps concernées, y compris éventuellement le cuir chevelu, une composition cosmétique telle que définie dans la revendication 10.

**Revendications pour l'Etat contractant suivant : ES**

1. Composition cosmétique ou hygiénique, caractérisée par le fait qu'elle comprend comme ingrédient actif, notamment tensioactif et/ou hydratant, au moins un dérivé de dipeptide à groupement uréthanne de formule générale I :

$$R'O\text{-}CO\text{-}NH\text{-}CH_2\text{-}CO\text{-}NH\text{-}CH(R)\text{-}COOH \qquad (I)$$

dans laquelle R représente un groupement -H ou un groupement $-CH_2OH$,
et R' représente un groupement alkyle linéaire ou ramifié, éventuellement insaturé, ayant de 8 à 16 atomes de carbone,
ou un sel de dérivé de formule I, ou un mélange de dérivé de formule I et/ou de leurs sels, dans un véhicule compatible avec l'administration sur la peau et/ou sur les cheveux, ou compatible avec l'application dans les soins d'hygiène bucco-dentaire.

2. Composition selon la revendication 1, caractérisée par le fait que lesdits sels sont des sels métalliques ou des sels de cations organiques compatibles avec une application cosmétique, pharmaceutique ou alimentaire.

3. Composition selon la revendication 2, caractérisée par le fait que lesdits sels sont choisis parmi les sels de sodium, de zinc, de magnésium et d'aluminium, et les sels cuivriques.

4. Composition selon la revendication 2, caractérisée par le fait que lesdits sels sont des sels d'ammonium quaternaires.

5. Composition selon la revendication 4, caractérisée par le fait que lesdits sels d'ammonium quaternaires sont des sels d'un cation de formule

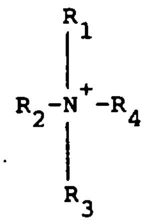

dans laquelle $R_1$, $R_2$, $R_3$ et $R_4$ représentent indépendamment un groupement $-CH_3$, $-CH_2-C_6H_5$ ou $-CH_2-CH_2OH$.

**6.** Composition selon l'une quelconque des revendications précédentes, caractérisée par le fait que R' est un groupement alkyle ayant 8 à 16 atomes de carbone.

**7.** Composition selon l'une quelconque des revendications précédentes, caractérisée par le fait qu'ils sont choisis parmi :

- la N-dodécyloxycarbonyl-glycyl-(D,L) sérine
- la N-dodécyloxycarbonyl-glycyl-glycine
- la N-éthyl-2-hexyloxycarbonyl-glycyl-glycine;
- la N-tétradécyloxycarbonyl-glycyl-glycine;
- la N-hexadécyloxycarbonyl-glycyl-glycine;

ainsi que leurs sels.

**8.** Composition selon l'une quelconque des revendications précédentes, caractérisée par le fait que la concentration dudit dérivé de dipeptide est dans la gamme de 0,05 à 20 % en poids, par rapport au poids total de la composition.

**9.** Composition selon la revendication 8, caractérisée par le fait que ladite concentration est dans la gamme 0,5 à 10 % en poids par rapport au poids total de la composition.

**10.** Procédé de préparation d'un dérivé de formule I tel que défini dans l'une quelconque des revendications précédentes, ou de ses sels, caractérisé par le fait que :

a) soit on fait réagir un composé de formule III :

$$R'O-CO-NH-CH_2-CO-O-CO-OR'' \quad (III)$$

dans laquelle R' est défini comme précédemment et R" représente un groupement éthyle ou isopropyle, avec un sel de glycine ou de sérine pour former un dérivé de formule I correspondant ;
b) soit on fait réagir un sel de diglycine avec un chloroformiate de formule IV :

$$R'-O-CO-Cl \quad (IV)$$

pour former un dérivé de formule I correspondant (avec R=H) ;
c) et que, si désiré, on transforme selon les méthodes connues ledit dérivé de formule I obtenu en sel correspondant.

**11.** Utilisation d'au moins un dérivé de formule I, tel que défini dans l'une quelconque des revendications 1 à 7, comme agent tensioactif.

**12.** Utilisation d'un dérivé de formule I tel que défini dans l'une quelconque des revendications 1 à 7 comme ingrédient actif dans la préparation d'une composition cosmétique ou pharmaceutique destinée au traitement ou au soin des peaux sèches.

**13.** Procédé de traitement cosmétique destiné notamment à améliorer l'aspect et l'élasticité de la peau des personnes à peau sèche, ou destiné à prévenir l'apparition des troubles esthétiques provoqués par ce phénomène de peau sèche, caractérisé par le fait que l'on applique sur la peau des parties du corps concernées, y compris éventuellement le cuir chevelu, une composition cosmétique telle que définie dans l'une quelconque des revendications 1 à 9.

## Patentansprüche

### Patentansprüche für folgende Vertragsstaaten : AT, BE, CH, LI, DE, DK, ES, FR, GB, GR, IT, NL, SE, PT

**1.** Dipeptidderivate mit Urethangruppierung gemäß allgemeiner Formel I:

$$R'O\text{-}CO\text{-}NH\text{-}CH_2\text{-}CO\text{-}NH\text{-}CH(R)\text{-}COOH \qquad (I)$$

worin R Wasserstoff oder die $-CH_2OH$-Gruppe, und
R' einen gerad- oder verzweigtkettigen, gegebenenfalls ungesättigten Alkylrest mit 8 bis 16 Kohlenstoffatomen bedeuten,
sowie die Salze der Derivate gemäß Formel I und Gemische von Derivaten gemäß Formel I und/oder deren Salze.

**2.** Derivate gemäß Anspruch 1, dadurch gekennzeichnet, daß die Salze in Form von Metallsalzen oder Salzen organischen Kationen vorliegen, die mit einer kosmetischen, pharmazeutischen oder im Zusammenhang mit der Ernährung stehenden Anwendung vereinbar sind.

**3.** Derivate gemäß Anspruch 2, dadurch gekennzeichnet, daß die Salze unter Natrium-, Zink-, Magnesium- und Aluminiumsalzen sowie unter Kupfersalzen ausgewählt sind.

**4.** Derivate gemäß Anspruch 2, dadurch gekennzeichnet, daß die Salze quaternäre Ammoniumsalze darstellen.

**5.** Derivate gemäß Anspruch 4, dadurch gekennzeichnet, daß die quaternären Ammoniumsalze solche eines Kations gemäß Formel

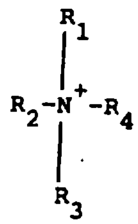

darstellen, worin
$R_1$, $R_2$, $R_3$ und $R_4$ unabhängig voneinander eine $-CH_3$-, $-CH_2\text{-}C_6H_5$- oder $-CH_2\text{-}CH_2OH$-Gruppe bedeuten.

**6.** Derivate gemäß einem der vorstehenden Ansprüche, dadurch gekennzeichnet, daß R' einen Alkylrest mit 8 bis 16 Kohlenstoffatomen bedeutet.

**7.** Derivate gemäß einem der vorstehenden Ansprüche, dadurch gekennzeichnet, daß sie unter den folgenden Verbindungen ausgewählt sind:

- N-Dodecyloxycarbonyl-glycyl-(D,L)-serin
- N-Dodecyloxycarbonyl-glycyl-glycin
- N-Ethyl-2-hexyloxycarbonyl-glycyl-glycin
- N-Tetradecyloxycarbonyl-glycyl-glycin
- N-Hexadecyloxycarbonyl-glycyl-glycin

sowie deren Salze.

**8.** Verfahren zur Synthese eines Derivats einer Struktur, deren Bedeutung in einem der vorstehenden Ansprüche angegeben ist, dadurch gekennzeichnet, daß man entweder

(a) eine Verbindung gemäß Formel III

$$R'O\text{-}CO\text{-}NH\text{-}CH_2\text{-}CO\text{-}O\text{-}CO\text{-}OR'' \qquad (III),$$

worin R' die vorstehende Bedeutung aufweist, und R" eine Ethyl- oder Isopropylgruppe bedeutet,

zusammen mit einem Glycin- oder Serinsalz zur Bildung eines Derivats gemäß der entsprechenden Formel I reagieren läßt, oder daß man

(b) ein Diglycinsalz mit einem Chlorformiat gemäß Formel IV

$$R'\text{-}O\text{-}CO\text{-}Cl \qquad (IV)$$

zur Bildung eines entsprechenden Derivats gemäß Formel I (wobei R = H bedeutet) miteinander reagieren läßt, und daß man

(c) gegebenenfalls mittels bekannter Verfahren das erhaltene Derivat gemäß Formel I zu dem entsprechenden Salz umsetzt.

**9.** Verwendung mindestens eines Dipeptidderivats, dessen Struktur die in einem der Ansprüche 1 bis 7 angegebene Bedeutung aufweist, als Tensid.

**10.** Kosmetische oder hygienische Zubereitung oder Arzneimittel, dadurch gekennzeichnet, daß das Mittel jeweils als Wirkstoff, insbesondere als Tensid und/oder Hydratisierungsmittel mindestens ein Dipeptidderivat, das die in einem der Ansprüche 1 bis 7 angegebene Bedeutung hat, in einem mit der Applikation auf der Haut tolerierbaren Träger und/oder mit den Haaren annehmbaren Träger enthält, oder einem Träger, der mit der Mund- oder Zahnhygiene vereinbar ist.

**11.** Mittel gemäß Anspruch 10, dadurch gekennzeichnet, daß die Konzentration an dem Dipeptidderivat in dem Bereich von 0,05 bis 20 Gew.-% in bezug auf das Gesamtgewicht der Zusammensetzung liegt.

**12.** Mittel gemäß Anspruch 11, dadurch gekennzeichnet, daß die Konzentration in dem Bereich von 0,5 bis 10 Gew.-% in bezug auf das Gesamtgewicht der Zusammensetzung liegt.

**13.** Verwendung eines Dipeptidderivats, das die in einem der Ansprüche 1 bis 7 angegebene Bedeutung aufweist, als Wirkstoff bei der Herstellung einer kosmetischen Zusammensetzung oder eines Arzneimittels, wobei das Mittel jeweils zur Behandlung oder der Pflege trockener Haut bestimmt ist.

**14.** Verfahren zur kosmetischen Behandlung, die insbesondere dazu vorgesehen ist, das Aussehen und die Elastizität der Haut von Personen mit trockener Haut zu verbessern, oder dafür vorgesehen ist, die ästhetischen Probleme im Zusammenhang mit dem Aussehen der Haut zu verhüten, welche auf dem Phänomen der trockenen Haut beruhen, dadurch gekennzeichnet, daß auf der Haut an den betroffenen Körperstellen einschließlich gegebenenfalls den behaarten Stellen am Kopf, eine kosmetische Zubereitung zur Anwendung bringt, wie sie im Anspruch 10 gekennzeichnet ist.

**Patentansprüche für folgenden Vertragsstaat : ES**

**1.** Kosmetische oder hygienische Zubereitung, dadurch gekennzeichnet, daß sie als Wirkstoff, insbesondere als Tensid und/ oder Hydratisierungsmittel mindestens ein Dipeptidderivat mit Urethangruppierung gemäß allgemeiner Formel I enthält:

$$R'O\text{-}CO\text{-}NH\text{-}CH_2\text{-}CO\text{-}NH\text{-}CH(R)\text{-}COOH \qquad (I)$$

worin R Wasserstoff oder die $-CH_2OH$-Gruppe, und
R' einen gerad- oder verzweigtkettigen, gegebenenfalls ungesättigten Alkylrest mit 8 bis 16 Kohlenstoffatomen bedeuten, oder ein Salz des Derivats gemäß Formel I oder ein Gemisch von Derivaten gemäß Formel I und/oder deren Salze in einem Träger, der mit der Anwendung auf der Haut und/oder an den Haaren oder im Zusammenhang mit der Anwendung zur Mund- und Zahnpflege vereinbar ist.

**2.** Mittel gemäß Anspruch 1 , dadurch gekennzeichnet, daß die Salze in Form von Metallsalzen oder Salzen organischen Kationen vorliegen, die mit einer kosmetischen, pharmazeutischen oder im Zusammenhang mit der Ernährung stehenden Anwendung vereinbar sind.

**3.** Mittel gemäß Anspruch 2, dadurch gekennzeichnet, daß die Salze unter Natrium-, Zink-, Magnesium- und Aluminiumsalzen sowie unter Kupfersalzen ausgewählt sind.

**4.** Mittel gemäß Anspruch 2, dadurch gekennzeichnet, daß die Salze quaternäre Ammoniumsalze darstellen.

**5.** Mittel gemäß Anspruch 4, dadurch gekennzeichnet, daß die quaternären Ammoniumsalze solche eines Kations gemäß Formel

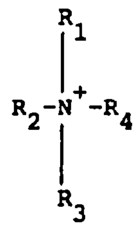

darstellen, worin
$R_1$, $R_2$, $R_3$ und $R_4$ unabhängig voneinander eine $-CH_3$-, $-CH_2-C_6H_5$- oder $-CH_2-CH_2OH$-Gruppe bedeuten.

**6.** Mittel gemäß einem der vorstehenden Ansprüche, dadurch gekennzeichnet, daß R' einen Alkylrest mit 8 bis 16 Kohlenstoffatomen bedeutet.

**7.** Mittel gemäß einem der vorstehenden Ansprüche, dadurch gekennzeichnet, daß (der Wirkstoff oder die Wirkstoffe) unter den folgenden Verbindungen ausgewählt sind:

- N-Dodecyloxycarbonyl-glycyl-(D,L)-serin
- N-Dodecyloxycarbonyl-glycyl-glycin
- N-Ethyl-2-hexyloxycarbonyl-glycyl-glycin
- N-Tetradecyloxycarbonyl-glycyl-glycin
- N-Hexadecyloxycarbonyl-glycyl-glycin

sowie deren Salze.

**8.** Mittel gemäß einem der vorstehenden Ansprüche, dadurch gekennzeichnet, daß die Konzentration an dem Dipeptidderivat in dem Bereich von 0,05 bis 20 Gew.-% in bezug auf das Gesamtgewicht der Zusammensetzung liegt.

**9.** Mittel gemäß Anspruch 8, dadurch gekennzeichnet, daß die Konzentration in dem Bereich von 0,5 bis 10 Gew.-% in bezug auf das Gesamtgewicht der Zusammensetzung liegt.

**10.** Verfahren zur Herstellung eines Derivats gemäß Formel I, das die in einem der vorstehenden Ansprüche angegebene Bedeutung aufweist, oder deren Salze, dadurch gekennzeichnet, daß man entweder

(a) eine Verbindung gemäß Formel III

$$R'O-CO-NH-CH_2-CO-O-CO-OR'' \quad (III),$$

worin R' die vorstehende Bedeutung aufweist, und R" eine Ethyl- oder Isopropylgruppe bedeutet, zusammen mit einem Glycin- oder Serinsalz zur Bildung eines Derivats gemäß der entsprechenden Formel I reagieren läßt, oder daß man

(b) ein Diglycinsalz mit einem Chlorformiat gemäß Formel IV

$$R'-O-CO-Cl \quad (IV)$$

zur Bildung eines entsprechenden Derivats gemäß Formel I (wobei R = H bedeutet) miteinander reagieren läßt, und daß man

(c) gegebenenfalls mittels bekannter Verfahren das erhaltene Derivat gemäß Formel I zu dem entsprechenden Salz umsetzt.

**11.** Verwendung mindestens eines Derivats gemäß Formel I, dessen Struktur die in einem der Ansprüche 1 bis 7 angegebene Bedeutung aufweist, als Tensid.

**12.** Verwendung eines Derivats gemäß Formel I, das jeweils die in einem der Ansprüche 1 bis 7 angegebene Bedeutung aufweist, als Wirkstoff bei der Herstellung einer kosmetischen Zubereitung oder eines Arzneimittels, wobei die Zusammensetzung jeweils zur Behandlung oder der Pflege trockener Haut bestimmt ist.

**13.** Verfahren zur kosmetischen Behandlung, die insbesondere dafür vorgesehen ist, das Aussehen und die Elastizität der Haut von Personen mit trockener Haut zu verbessern, oder dafür vorgesehen ist, das Erscheinen von unästhetischen Symptomen zu verhüten, welche auf dem Phänomen der trockenen Haut beruhen, dadurch gekennzeichnet, daß auf den betroffenen Körperstellen der Haut, gegebenenfalls einschließlich der behaarten Stellen am Kopf, eine kosmetische Zubereitung zur Anwendung bringt, wie sie in einem der Ansprüche 1 bis 9 gekennzeichnet ist.

**Claims**

**Claims for the following Contracting States : AT, BE, CH, LI, DE, DK, ES, FR, GB, GR, IT, NL, SE, PT**

**1.** Dipeptide derivatives containing a urethane group, of general formula I:

$$R'O\text{-}CO\text{-}NH\text{-}CH_2\text{-}CO\text{-}NH\text{-}CH(R)\text{-}COOH \qquad (I)$$

in which R represents an -H group or a $-CH_2OH$ group,
and R' represents a linear or branched alkyl group, optionally unsaturated, having from 8 to 16 carbon atoms,
as well as the salts of the derivatives of formula I, and the mixtures of the derivatives of formula I and/or their salts.

**2.** Derivatives according to Claim 1, characterized in that the said salts are metal salts or salts of organic cations compatible with a cosmetic, pharmaceutical or food application.

**3.** Derivatives according to Claim 2, characterized in that the said salts are chosen from the sodium, zinc, magnesium and aluminium salts and the cupric salts.

**4.** Derivatives according to Claim 2, characterized in that the said salts are quaternary ammonium salts.

**5.** Derivatives according to Claim 4, characterized in that the said quaternary ammonium salts are salts of a cation of formula

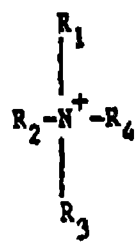

in which $R_1$, $R_2$, $R_3$ and $R_4$ independently represent a $-CH_3$, $-CH_2\text{-}C_6H_5$ or $-CH_2\text{-}CH_2OH$ group.

**6.** Derivatives according to any one of the preceding claims, characterized in that R' is an alkyl group having 8 to 16 carbon atoms.

**7.** Derivatives according to any one of the preceding claims, characterized in that they are chosen from:

- N-(dodecyloxycarbonyl)glycyl-DL-serine;
- N-(dodecyloxycarbonyl)glycylglycine;
- N-(2-ethylhexyloxycarbonyl)glycylglycine;
- N-(tetradecyloxycarbonyl)glycylglycine;
- N-(hexadecyloxycarbonyl)glycylglycine;

as well as their salts.

**8.** Process for preparing a derivative as defined in any one of the preceding claims, characterized in that:

a) either a compound of formula III:

$$R'O\text{-}CO\text{-}NH\text{-}CH_2\text{-}CO\text{-}O\text{-}CO\text{-}OR'' \qquad (III)$$

in which R' is defined as above and R" represents an ethyl or isopropyl group,
is reacted with a glycine or serine salt to form a corresponding derivative of formula I;
b) or a diglycine salt is reacted with a chloroformate of formula IV:

$$R'\text{-}O\text{-}CO\text{-}Cl \qquad (IV)$$

to form a corresponding derivative of formula I (with R=H);
c) and in that, if desired, the said derivative of formula I obtained is converted according to known methods to a corresponding salt.

**9.** Use of at least one dipeptide derivative as defined in any one of Claims 1 to 7 as a surfactant.

**10.** Cosmetic, hygiene or pharmaceutical composition, characterized in that it comprises as active, in particular surfactant and/or hydrating, ingredient at least one dipeptide derivative as defined in any one of Claims 1 to 7, in a vehicle compatible with administration to the skin and/or hair or compatible with application in dentibuccal hygiene care.

**11.** Composition according to Claim 10, characterized in that the concentration of the said dipeptide derivative is within the range 0.05 to 20 % by weight relative to the total weight of the composition.

**12.** Composition according to Claim 11, characterized in that the said concentration is within the range 0.5 to 10 % by weight relative to the total weight of the composition.

**13.** Use of a dipeptide derivative as defined in any one of Claims 1 to 7, as active ingredient in the preparation of a cosmetic or pharmaceutical composition intended for the treatment or care of dry skins.

**14.** Cosmetic treatment process intended, in particular, for improving the appearance and elasticity of the skin of individuals having dry skin, or intended for preventing the onset of an unsightly appearance caused by this phenomenon of dry skin, characterized in that a cosmetic composition as defined in Claim 10 is applied to the skin of the parts of the body affected, where appropriate including the scalp.

**Claims for the following Contracting State : ES**

**1.** Cosmetic or hygiene composition, characterized in that it comprises as active, in particular surfactant and/or hydrating, ingredient at least one dipeptide derivative containing a urethane group, of general formula I:

$$R'O\text{-}CO\text{-}NH\text{-}CH_2\text{-}CO\text{-}NH\text{-}CH(R)\text{-}COOH \qquad (I)$$

in which R represents an -H group or a $-CH_2OH$ group,
and R' represents a linear or branched alkyl group, optionally unsaturated, having from 8 to 16 carbon atoms,
or a salt of a derivative of formula I, or a mixture of a derivative of formula I and/or its salts, in a vehicle compatible with administration to the skin and/or hair or compatible with application in dentibuccal hygiene care.

**2.** Composition according to Claim 1, characterized in that the said salts are metal salts or salts of organic cations compatible with a cosmetic, pharmaceutical or food application.

**3.** Composition according to Claim 2, characterized in that the said salts are chosen from the sodium, zinc, magnesium and aluminium salts and the cupric salts.

**4.** Composition according to Claim 2, characterized in that the said salts are quaternary ammonium salts.

**5.** Composition according to Claim 4, characterized in that the said quaternary ammonium salts are salts of a cation of formula

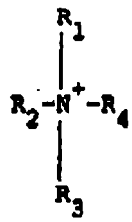

in which $R_1$, $R_2$, $R_3$ and $R_4$ independently represent a $-CH_3$, $-CH_2-C_6H_5$ or $-CH_2-CH_2OH$ group.

**6.** Composition according to any one of the preceding claims, characterized in that R' is an alkyl group having 8 to 16 carbon atoms.

**7.** Composition according to any one of the preceding claims, characterized in that it is chosen from:

- N-(dodecyloxycarbonyl)glycyl-DL-serine;
- N-(dodecyloxycarbonyl)glycylglycine;
- N-(2-ethylhexyloxycarbonyl)glycylglycine;
- N-(tetradecyloxycarbonyl)glycylglycine;
- N-(hexadecyloxycarbonyl)glycylglycine;

as well as its salts.

**8.** Composition according to any one of the preceding claims, characterized in that the concentration of the said dipeptide derivative is within the range 0.05 to 20 % by weight relative to the total weight of the composition.

**9.** Composition according to Claim 8, characterized in that the said concentration is within the range 0.5 to 10 % by weight relative to the total weight of the composition.

**10.** Process for preparing a derivative of formula I as defined in any one of the preceding claims, or its salts, characterized in that:

a) either a compound of formula III:

$$R'O-CO-NH-CH_2-CO-O-CO-OR" \qquad (III)$$

in which R' is defined as above and R" represents an ethyl or isopropyl group,
is reacted with a glycine or serine salt to form a corresponding derivative of formula I;
b) or a diglycine salt is reacted with a chloroformate of formula IV:

$$R'-O-CO-Cl \qquad (IV)$$

to form a corresponding derivative of formula I (with R=H);
c) and in that, if desired, the said derivative of formula I obtained is converted according to known methods to a corresponding salt.

**11.** Use of at least one derivative of formula I as defined in any one of Claims 1 to 7, as a surfactant.

**12.** Use of a derivative of formula I as defined in any one of Claims 1 to 7, as active ingredient in the preparation of a cosmetic or pharmaceutical composition intended for the treatment or care of dry skins.

**13.** Cosmetic treatment process intended, in particular, for improving the appearance and elasticity of the skin of individuals having dry skin, or intended for preventing the onset of an unsightly appearance caused by this phenomenon of dry skin, characterized in that a cosmetic composition as defined in any one of Claims 1 to 9 is applied to the skin of the parts of the body affected, where appropriate including the scalp.